# EUROPEAN PATENT SPECIFICATION

(45) Date of publication of patent specification: **27.07.94**   (51) Int. Cl.5: **C07H 15/04**, C07H 3/04, C07H 13/06

(21) Application number: **86309325.8**

(22) Date of filing: **28.11.86**

The file contains technical information submitted after the application was filed and not included in this specification

(54) **Process for preparing derivatives of maltose.**

(30) Priority: **30.11.85 JP 270253/85**

(43) Date of publication of application:
**16.06.87 Bulletin 87/25**

(45) Publication of the grant of the patent:
**27.07.94 Bulletin 94/30**

(84) Designated Contracting States:
**DE FR GB**

(56) References cited:

**CEREAL SCIENCE TODAY, vol. 17, no. 7, July 1972, pages 180-188, AmericanAssociation of Cereal Chemists, Inc., St. Paul, Minnesota, US; J.E. HODGES etal.: "Useful properties of maltose"**

**W. PIGMAN et al.: "The carbohydrates, chemistry and biochemistry, vol. 1B, 2ndedition, 1980, page 1466, Academic Press, New York, US**

(73) Proprietor: **KABUSHIKI KAISHA HAYASHIBARA SEIBUTSU KAGAKU KEN-KYUJO**
**2-3, 1-chome, Shimoishii**
**Okayama-shi Okayama(JP)**

(72) Inventor: **Mitsuhashi, Masakazu**
**4-11, 1-chome, Kobashi-cho**
**Okayama-shi Okayama(JP)**
Inventor: **Sakai, Shuzo**
**3-41, 1-chome, Ejiri-asahigaoka, Seto-cho**
**Akaiwa-gun Okayama(JP)**
Inventor: **Miyake, Toshio**
**7-10-403, 1-chome, Okuda**
**Okayama-shi Okayama(JP)**

(74) Representative: **Pendlebury, Anthony et al**
**PAGE, WHITE & FARRER**
**54 Doughty Street**
**London WC1N 2LS (GB)**

THE JOURNAL OF ORGANIC CHEMISTRY, vol. 31, no. 11, November 1966, pages 3704-3707; I.M.E. THIEL et al.: "The reaction of ammonia with acylated disaccharides. VIII. Octa-O-benzoylmaltose and benzoyl derivatives of maltose"

CHEMICAL AND PHARMACEUTICAL BULLE-TIN, vol. 29, no. 2, February 1981, pages 505-512; Y. NISHIKAWA et al.: "Chemical and biochemical studies on carbohydrateesters. IX. Antitumor effects of selectively fatty acylated products ofmaltose"

**Description**

The present invention relates to a process for preparing maltose derivatives.

In the specification, percentages and parts will be expressed by weight based on a dry solids basis, unless specified otherwise.

Maltose derivatives such as esters, ethers, halides, nitrogen-atom- or sulfur-atom-containing derivatives and glycosides are now in great demand because such derivatives are useful in or as food additives, detergents, pharmaceuticals, synthetic intermediates and substrates for enzymes.

For example, fatty acid esters of maltose are found to be attractive as safe- and highly-active surfactants while in contrast sucrose esters have unsatisfactory solubility and emulsion forming ability, and also have undesirable physiological effects.

Mono- or poly-fatty acid esters of maltose, obtained by allowing maltose to react with a fatty acid, such as stearic acid, palmitic acid and myristic acid, in a mixture of hydrochloric acid and pyridine, are promising anti-tumour or anti-cancer agents because, as reported by Y. Nishikawa et al., Chemical and Pharmaceutical Bulletin, Vol. 29, No. 2, pp. 505-513 (1981), the fatty acid esters show an antioncotic activity.

Maltose (lower alkyl) ethers are useful as synthetic intermediates; while maltose ethers having higher alkyl groups of 8-25 carbon atoms are promising safe- and highly-active surfactants for detergents.

As disclosed in Japanese Patent Laid-Open Nos. 5,199/84 and 48,992/85, and R. Khan, Advances in Carbohydrate Chemistry and Biochemistry, Vol. 39, pp. 213-278 (1981), such maltose derivatives are generally prepared by allowing an anhydrous maltose to react with a suitable reagent under anhydrous conditions; the yield and quality of the maltose derivatives vary with the removal of moisture from the reaction system.

Since maltose, however, generally contains 5.5-6.5% of moisture, its use inevitably leads to unsatisfactory results.

For this reason, maltose is dried to anhydrousness prior to chemical reaction. Since maltose is highly hygroscopic, and the water of crystallisation in beta-maltose hydrate can not be easily removed, incorporation of moisture into a reaction system is inevitable even when the reaction is carefully controlled. Thus, it is very difficult to prepare high-quality maltose derivatives at a consistently high yield.

Chem. Pharm. Bul. 29(2), 505-513 (1981) discloses fatty acylation of maltose. Beta-maltose hydrate is dehydrated to yield anhydrous maltose which is then reacted with an acid chloride in pyridine. A crude product is produced requiring chromatography to resolve the desired maltose esters. The esters were used in the study of anti-tumour effects in mice.

We studied ways of preparing high-quality maltose derivatives in a high yield and low cost in order to overcome the said disadvantages of the conventional processes.

According to the present invention there is provided a process for preparing a derivative of maltose, the process comprising reacting maltose with a suitable reagent under anhydrous conditions to form the desired derivative, characterised in that said maltose is crystalline alpha-maltose, whereby the desired derivative is produced in an almost theoretical yield and high quality.

The present invention is based on the discovery that the stated disadvantages of the prior art can be overcome by allowing a crystalline alpha-maltose, preferably a crystalline alpha-maltose having an alpha-maltose isomer content of 55% or more, to react with a reagent under anhydrous conditions.

We have found that the drying step which is essential when reacting anhydrous amorphous maltose or crystalline beta-maltose can be very much shortened or even omitted because, unlike anhydrous amorphous maltose or crystalline beta-maltose hydrate, crystalline alpha-maltose contains no water of crystallisation and is substantially non-hygroscopic; and because the equilibrium water can be easily removed from crystalline alpha-maltose.

We have also found that the favourable properties of crystalline alpha-maltose such as high-fluidity, high affinity for organic solvents, and, occasionally, high solubility in the solvents, very much facilitate the production steps.

We found that, in certain esterifications or etherifications, the use of crystalline alpha-maltose leads to maltose esters and ethers in the theoretical yield.

The term "maltose derivative(s)" used throughout this specification is used to mean maltose derivatives which can be prepared by the chemical reaction of maltose with a suitable reagent (as hereinafter defined) under anhydrous conditions, wherein the water in the reaction system lowers the reactivity of a reagent, causes an undesirable side reaction and/or inhibits the main reaction to lower the yield and quality of the product and/or to increase its production cost.

Examples of such maltose derivative are esters, ethers, halides, nitrogen-atom- or sulfur-atom-containing derivatives and carboxyl derivatives of maltose as described, for example, in R. Khan, Advances in Carbohydrate Chemistry and Biochemistry, Vol. 39, pp. 213-278 (1981), or G.R. Ames, Chemical Reviews, Vol. 60, pp. 541 (1960).

The term "reagent" used throughout this specification means one or more reactive agents which give such maltose derivatives when reacted with crystalline alpha-maltose under anhydrous conditions, for example, an acid, base, alcohol, aldehyde, ketone, halogen, amine, cyanide, nitrile, oxirane, isocyanate or a reactive derivative thereof.

The crystalline alpha-maltose which is preferred for use in the invention is that in pulverulent form.

We have found that a high-purity maltose having a maltose content of 85% or more is preferable as the starting material maltose for preparing crystalline alpha-maltose.

Such high-purity maltose may be a commercially available crystalline beta-maltose hydrate or that prepared by saccharifying starch in the usual manner.

A high-purity maltose may be prepared from starch, for example, by subjecting a gelatinized- or liquefied-starch to the action of beta-amylase in a manner as disclosed, for example, in Japanese Patent Publication No. 11,437/81 or 17,078/81.

High purity maltose can also be prepared by the method disclosed, for example, in Japanese Patent Publication No. 11,437/81 or 17,078/81, wherein a gelatinized- or liquefied-starch is subjected to beta-amylase to form maltose which is then separated from the dextrin polymers; or the method disclosed, for example, in Japanese Patent Publication No. 13,089/72 or 3,938/79, wherein a gelatinized- or liquefied-starch is subjected to the actions of a debranching enzyme, such as isoamylase or pullulanase, and beta-amylase, followed by recovery of a high-purity maltose.

It is advantageous to augment the maltose purity in the obtained high-purity maltose by use of a method in which other saccharides such as maltotriose are converted into maltose with an enzyme as disclosed, for example, in Japanese Patent Publication No. 28,153/81, 3,356/82 or 28,154/81; or removed by fractionation as disclosed, for example, in Japanese Patent Laid-Open No. 23,799/83, using a column of a strongly-acidic cation exchange resin.

Such fractionation can be effected by a fixed-bed-, moving-bed- or simulated-moving-bed-method.

In order to prepare a crystalline alpha-maltose from the high-purity maltose obtained in this way and having a maltose content of 85% or more, for example, the high-purity maltose is prepared into a syrup having a moisture content of about 10%, added with a crystalline alpha-maltose seed in an amount less than about 0.01-20%, and crystallized at about 40-140°C to obtain a massecuite having an alpha-maltose isomer content of over 48%, after which the massecuite is prepared, for example, into a powder, granules or into short cylindrical form, by conventional pulverization, ageing and/or drying, for example, crystal separation, block-pulverization, compression granulation, fluidized-bed granulation, and spray-drying. The obtained crystalline alpha-maltose is generally a substantially non-hygroscopic pulverulent solid with a moisture content of less than 1%. The melting point of crystalline alpha-maltose is 130°C or higher which is much higher than that of beta-maltose, i.e. 121-125°C. Specifically, the melting point of crystalline alpha-maltose having an alpha-maltose isomer content of 60% or higher is about 140°C or higher, and the crystalline alpha-maltose can be advantageously used for preparing maltose derivatives because of its non-hygroscopicity, non-adhesiveness and high-fluidity.

The reaction of crystalline alpha-maltose with a suitable reagent in order to prepare a desired maltose derivative is generally performed by dissolving or suspending crystalline alpha-maltose in an organic solvent such as methanol, ethanol, isopropyl alcohol, isoamyl alcohol, t-butyl alcohol, acetone, dimethylsulfoxide, acetonitrile, N,N-dimethylformamide, N-isopropylformamide, N-methylpyrrolidone, N-formylmorpholine, hexamethylphosphorictriamide, tetrahydrofuran, dioxane or pyridine, and the resultant solution or suspension is then added with the reagent and, if necessary, catalyst, and allowed to react under anhydrous conditions. Of course, the solvent may be totally or partially omitted when the reagent and/or catalyst per se acts as the solvent.

For example, organic acid esters of maltose, such as acetate and benzoate, can be prepared by allowing crystalline alpha-maltose to react with an acid anhydride in the presence of a basic catalyst, for example, pyridine or N,N-dimethylaniline.

Inorganic acid esters of maltose such as carbonate, sulfate and phosphorate can be prepared by reacting crystalline alpha-maltose with the respective acid halide in the presence of a basic catalyst, for example, pyridine or N,N-dimethylaniline.

Fatty acid esters can be prepared, for example, by heating a lower alkyl alcohol ester of fatty acid together with crystalline alpha-maltose in the presence of a basic catalyst such as an alkaline carbonate, alkaline hydroxide, alkaline alcoholate or quaternary amine alcoholate to effect an ester exchange reaction while removing the resultant lower alkyl alcohol from the reaction system; or by allowing crystalline alpha-maltose to react with a corresponding esterifying reagent, such as a fatty acid halide, thiazolithionic fatty acid amide or p-nitrophenyl fatty acid ester, in the presence of a basic catalyst. Fatty acid esters which are promising food additives, hyperalimentations or anti-tumour agents, such as maltose laurate, maltose oleate, maltose stearate, maltose linoleate, maltose linolenate, maltose palmitate, and maltose myristate, can be prepared in this way.

Maltose ethers which are useful as synthetic intermediates or detergents can be prepared by heating crystalline alpha-maltose together with an excessive amount of an alcohol having an appropriate chain length in the presence of a catalyst such as sulfuric acid, hydrochloric acid, phosphoric acid, phosphorous acid, p-toluenesulfonic acid or boron trifluoride while removing the resultant water from the reaction system.

Halides or sulfur-atom-containing derivatives which are useful as synthetic intermediates can be prepared respectively by effecting halogenation using p-sulfuryl chloride or p-toluenesulfonylation using a phosphorus-atom-containing reagent under anhydrous conditions.

The present invention also facilitates the protection of a hydroxyl or active aldehyde group in the maltose molecule during conversion into a useful maltose derivative as in the case of trimethylsilylation prior to the analysis of maltose.

The present invention will be illustrated further by the following Experiments.

Experiment 1

Comparison of several maltose materials

Several starch sugar products as listed in Table I, commercialized by Hayashibara Co., Ltd., Okayama, Japan, were used as the material. The syrup product, i.e. "MALSTAR" or "HM-75", was placed in an evaporator and evaporated in vacuo to give a moisture content of 4.5%. The pulverulent crystalline beta-maltose hydrate product, i.e. "SUNMALT", "MALTOSE H", "MALTOSE HH", or "MALTOSE HHH", was dissolved with a small amount of water while heating, placed in an evaporator, and evaporated in vacuo to give a moisture content of 4.5%.

The resultant syrup with a moisture content of about 4.5% was placed in a crystallizer. To the syrup was added 2% crystalline alpha-maltose seed which had been crystallized and recovered from an about 50% hot aqueous alcoholic solution of "MALTOSE HHH (a commercialized crystalline high-purity beta-maltose hydrate)", and the mixture was crystallized at 120°C for 20 minutes. Thereafter, the content was placed in an aluminum tray and aged at 90°C for 16 hours.

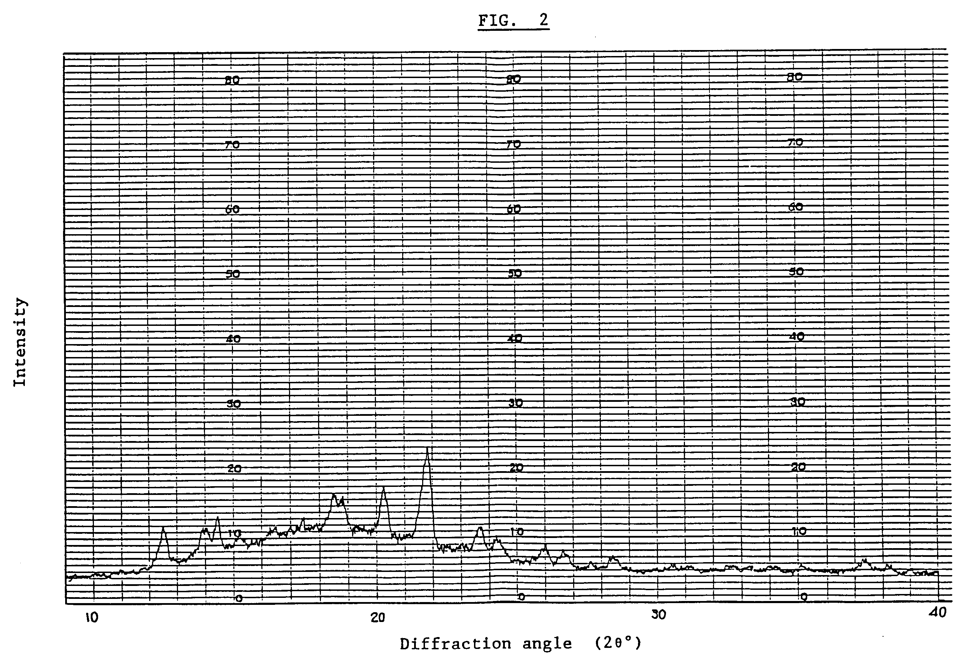

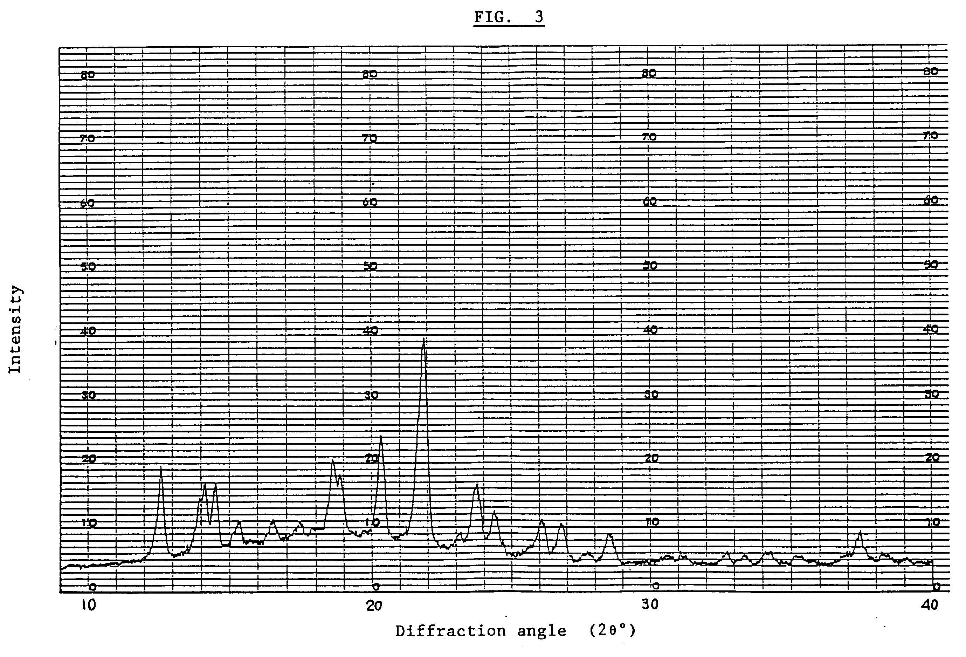

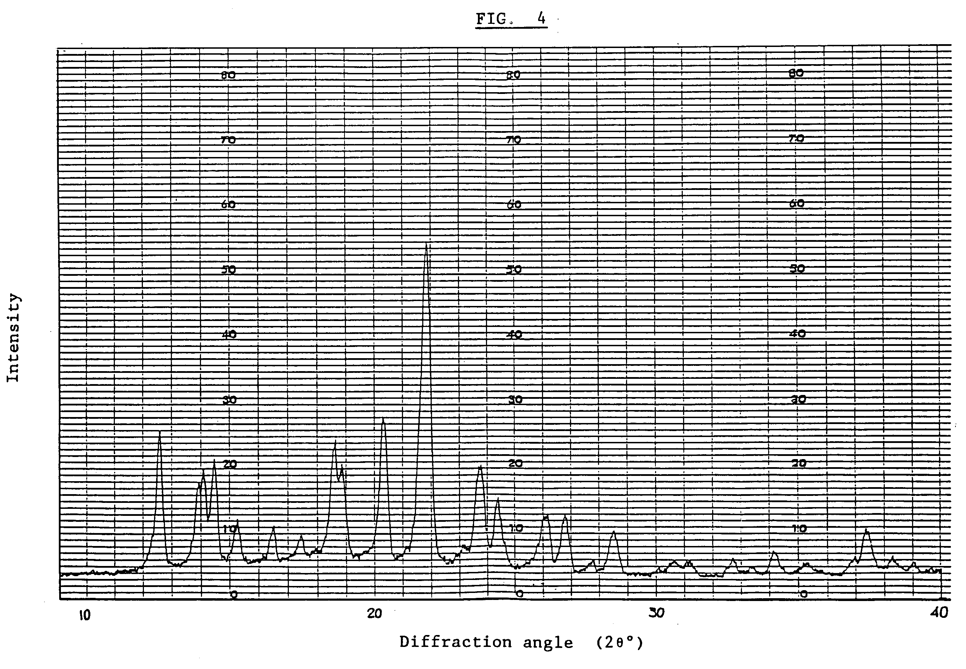

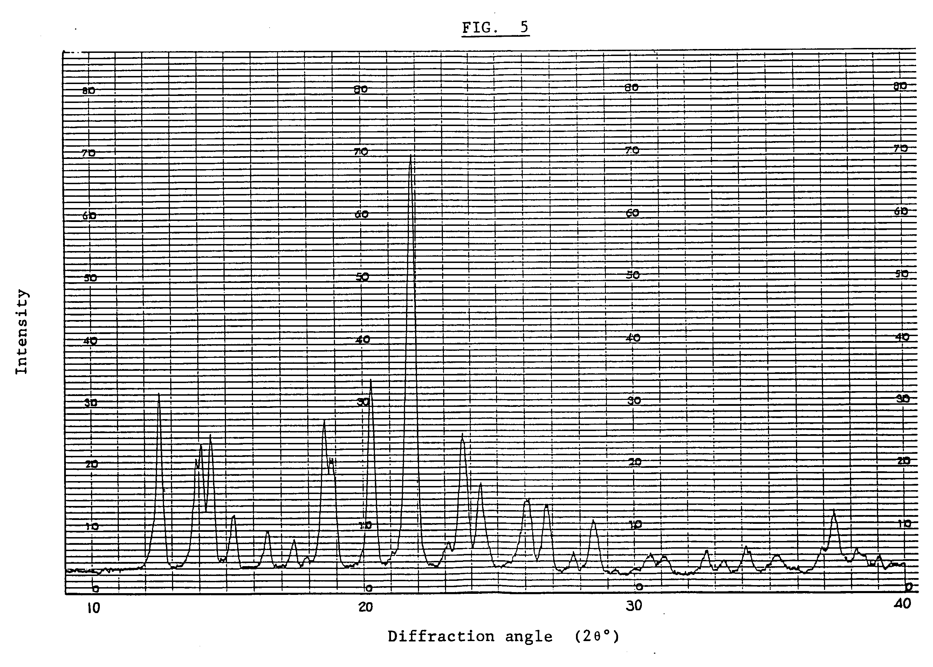

The resultant block was cooled to ambient temperature and finely divided. The alpha-maltose content in the resultant powder was determined by gas-chromatography as described by C.C. Sweeley et al., Journal of the American Chemical Society, Vol. 85, pp. 2497-2507 (1963). Separately, the powder was subjected to x-ray diffraction analysis using CuKα rays as described by F.H. Stodola et al., Journal of the American Chemical Society, Vol. 78, pp. 2514-2518 (1956) in order to check the presence of crystals. The x-ray diffraction apparatus employed was "GEIGERFLEX RAD-II B", commercialized by Rigaku Corporation, Chiyoda-ku, Tokyo, Japan. The results were as given in Table I. The x-ray diffraction patterns obtained are shown in Figures 1-5. Figure 1 shows the x-ray diffraction pattern of an anhydrous amorphous powder with an alpha-maltose content of 48%; Figure 2 shows the x-ray diffraction pattern of a crystalline powder with an alpha-maltose content of 55.6%; Figure 3 shows the x-ray diffraction pattern of a crystalline powder with an alpha-maltose content of 61.4%; Figure 4 shows the x-ray diffraction pattern of a crystalline powder with an alpha-maltose content of 68.7%; and Figure 5 shows an x-ray diffraction pattern of a crystalline powder with an alpha-maltose content of 74.2%.

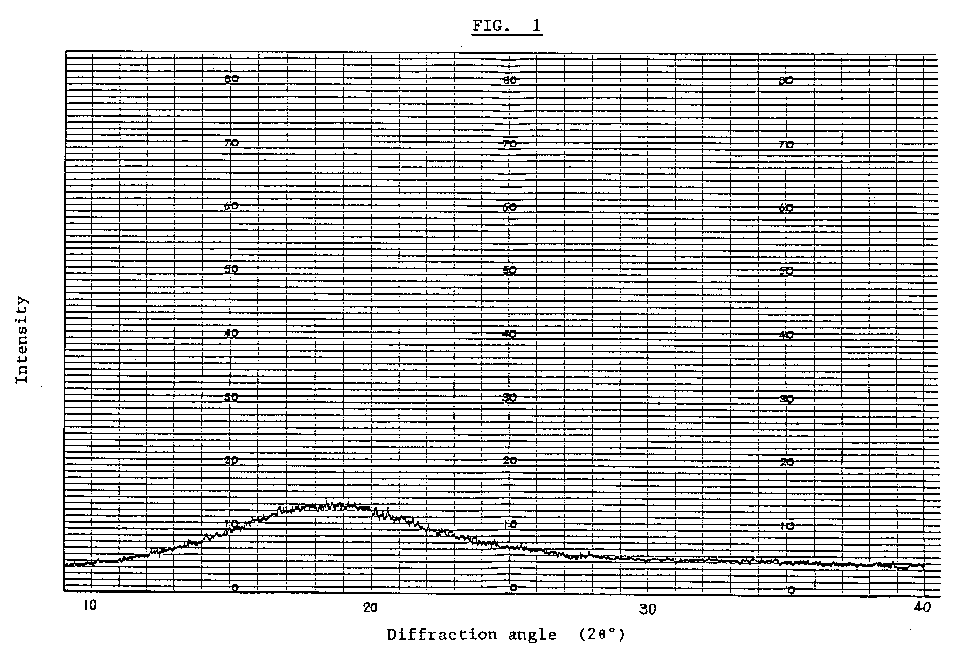

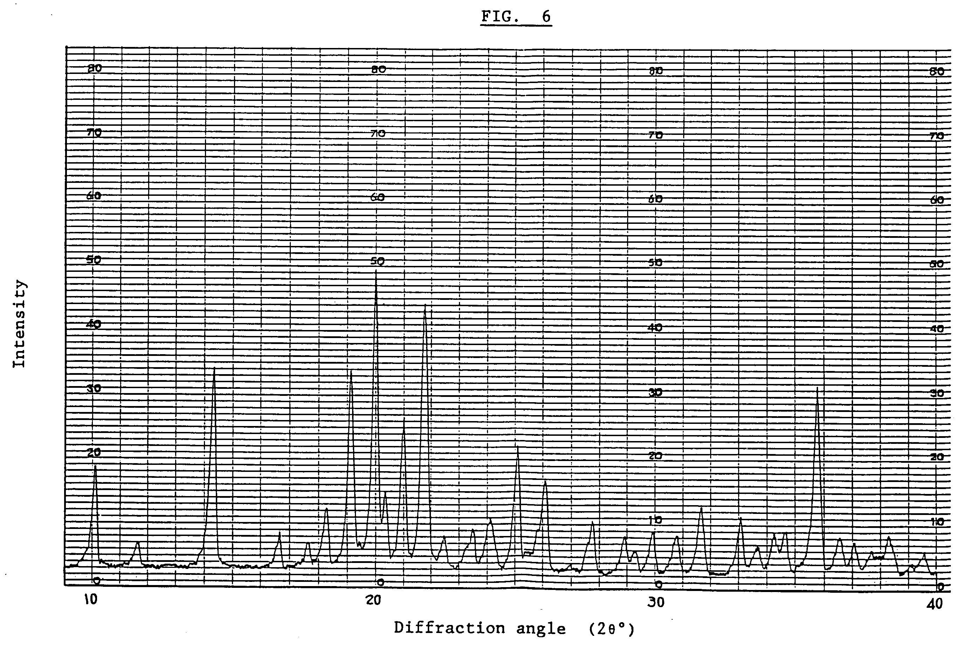

As a control, a portion of "MALTOSE HHH" was dissolved in water by heating, dried in vacuo, and finely divided to obtain an anhydrous amorphous powder which was then subjected to the x-ray diffraction analysis to obtain an x-ray diffraction pattern similar to that shown in Figure 1. The x-ray diffraction of "MALTOSE HHH" which was obtained is shown in Figure 6.

These x-ray diffraction results confirm that the alpha-maltose isomer content required for crystallization is 55% or higher, and that the maltose content of a maltose suitable as starting material is 85% or higher.

Table I

| Test No. | Starting Material maltose (Trade name) | Maltose content (%) | Alpha-maltose isomer content (%) | X-ray diffraction | |
|---|---|---|---|---|---|
| | | | | Crystal | Diffraction figure |
| 1 | MALSTAR® | 68.4 | 48.0 | Absent | FIG.1 |
| 2 | HM-75 | 79.6 | 48.0 | Absent | FIG.1 |
| 3 | SUNMALT® | 85.8 | 55.6 | Present | FIG.2 |
| 4 | MALTOSE H | 91.5 | 61.4 | Present | FIG.3 |
| 5 | MALTOSE HH | 96.2 | 68.7 | Present | FIG.4 |
| 6 | MALTOSE HHH | 99.7 | 74.2 | Present | FIG.5 |
| 7 | MALTOSE HHH | 99.7 | 48.0 | Absent | FIG.1 |
| 8 | MALTOSE HHH | 99.7 | 2.3 | Present | FIG.6 |

Experiment 2

Effect of starting material maltose on the formation of methyl maltoside

Five grams of a crystalline alpha-maltose specimen in Test No. 6, prepared by the method in Experiment 1, was refluxed for 18 hours together with 50 ml of 2.5% of hydrochloric acid in methanol while shutting off the moisture. At the end of the reaction, the amount of methyl maltoside in the reaction mixture was determined according to the high-pressure liquid chromatographic method as described in W.H. Cheethan and P. Sirimanne, Carbohydrate Research, Vol. 96, pp. 126-128 (1981).

The yield (%) of methyl maltoside was found about 65% as calculated by the total of alpha- and beta-methyl-maltosides against the theoretical yield based on the amount of maltose in the specimen used.

Thereafter, the remaining maltose and reagent were removed from the reaction mixture with a separatory funnel. Thus, methyl maltoside was obtained in the yield of about 62% from the resultant organic solution layer upon concentration, while from the aqueous layer about 27% of maltose was recovered intact.

The same experiment on anhydrous amorphous maltose in Test No. 8 gave similar results, while treatment of the methyl maltoside in the strongly coloured reaction mixture obtained by allowing "MALTOSE HHH" to react in the same manner gave a methyl maltoside yield of about 25% and maltose recovery yield of about 35%.

These results confirmed that, when using the crystalline alpha-maltose, the methyl matoside yield was extremely high and comparable to that attained with anhydrous amorphous maltose.

In contrast, when using crystalline beta-maltose hydrate, the reaction mixture was strongly coloured, and the methyl maltoside yield and maltose recovery yield were both inferior to those attained with crystalline alpha-maltose or anhydrous amorphous maltose.

Experiment 3

Effect of starting material maltose on the formation of octacetyl maltoside

A mixture solution of 50 g of acetic anhydride and a 65 g of dry pyridine was cooled to 0°C and added with 3 g of crystalline alpha-maltose specimen No. 6 in Experiment 1. The mixture was stirred at 0°C until the specimen was completely dissolved, and then allowed to stand for 18 hours at ambient temperature. The mixture was poured into ice water while stirring, allowed to stand briefly, and fed to a separatory funnel to obtain an organic layer which was then concentrated to obtain about 7 g of octacetyl maltoside.

It was found that octacetyl maltoside was recovered in an almost theoretical yield when calculated against the amount of maltose in the specimen used.

The experiment using anhydrous amorphous maltose in Test No. 8 gave similar results, while treatment of a reaction mixture obtained by allowing "MALTOSE HHH" to react in the same manner gave an octacetyl maltoside yield of only about 65%.

After crystalline alpha-maltose in Test No. 6 and anhydrous amorphous maltose in Test No. 8 were allowed first to stand for 2 hours at ambient temperature, then to react similarly as above, the crystalline alpha-maltose gave similar results, while the use of anhydrous amorphous maltose decreased the octacetyl maltoside yield to about 38%.

As is apparent from these data, when using crystalline alpha-maltose, the octacetyl maltoside yield is much higher than that attained with crystalline beta-maltose hydrate, and is comparable to the octacetyl maltoside yield attained with anhydrous amorphous maltose.

The reaction of crystalline alpha-maltose with a reagent according to the invention easily gives the same result as that attained with an anhydrous amorphous maltose prepared with great care.

Furthermore, the use of crystalline alpha-maltose is favourable in industrial-scale production of maltose derivative by the reaction with a reactive agent under anhydrous conditions due to the fact that crystalline alpha-maltose is substantially non-hygroscopic.

The following "Preparative Examples" illustrate the preparation of crystalline alpha-maltose.

Preparative Example 1

A suspension of 1 part of potato starch and 10 parts of water was added with a commercialized liquefying bacterial alpha-amylase, gelatinized by heating to 90°C, and immediately heated to 130°C to suspend the enzymatic reaction. Thus, a liquefied starch solution with a Dextrose Equivalent (DE) of about 0.5 was obtained. The starch solution was immediately cooled to 55°C, added with 100 units/g starch of isoamylase (EC 3.2.1.68) derived from a culture of Pseudomonas amyloderamosa ATCC 21262, and 50 units/starch of a soybean beta-amylase (EC 3.2.1.2), commercialized by Nagase & Company, Ltd., Osaka, Japan, under the trade name of "#1500", and saccharified at pH 5.0 for 40 hours to obtain a high-purity maltose solution with a maltose content of 92.5%, which was then decolourised with activated carbon, followed by purification and deionization with ion exchange resins. The maltose solution was concentrated to 75%, fed to a crystallizer, added with 1% crystalline beta-maltose monohydrate seed, adjusted to 40°C, and gradually cooled to 30°C in 2 days under gentle stirring conditions to obtain a crystal suspension. The crystals were separated from the suspension with a basket-type centrifuge, and washed by spraying a small amount of water to obtain a crystalline high-purity beta-maltose hydrate (purity 99.0%).

The high-purity maltose thus obtained was dissolved with a small amount of water while heating, placed in an evaporator, and evaported in vacuo to prepare a syrup with a moisture content of 5.5%. The content was fed to a crystallizer, added with 1% crystalline alpha-maltose seed obtained by the method in Test No. 6 in Experiment 1, crystallized at 100°C for 5 minutes while stirring, poured into a plastic tray, and aged at 70°C for 6 hours. The resultant block was then divided with a pulverizer, and dehydrated by the fluidized-bed drying to obtain a pulverulent crystalline alpha-maltose with an alpha-maltose isomer content of 73.3% in a yield of about 92% based on the material crystalline high-purity beta-maltose hydrate.

Preparative Example 2

An aqueous solution of a high-purity maltose having a maltose content of 92.5%, prepared by the method in Preparative Example 1, was concentrated in vacuo to give a moisture content of 20%, and sprayed with a high-pressure pump through a nozzle, equipped at the top of a spraying tower. Simultaneously, 100°C air was passed from the top of the tower towards a net conveyer carrying a fluidized crystalline alpha-maltose as the seed crystal, placed at the bottom of the tower, to collect the pulverized product on the net conveyer and also to fluidize the product out of the tower over a period of 60 minutes while passing an air stream of 70°C upwards through the net. The resultant product was then placed in an ageing tower and aged for 4 hours in an air stream of 70°C to obtain a pulverulent crystalline alpha-maltose with an alpha-maltose content of 66.2% in a yield of about 94% based on the material high-purity maltose.

Preparative Example 3

A suspension of 2 parts of corn starch and 10 parts of water was added with a commercialized bacterial liquefying alpha-amylase, gelatinized by heating to 90°C, and heated to 130°C to suspend the enzymatic reaction. The liquefied starch solution had a DE of about 2. The starch solution was immediately cooled to 55°C, added with 120 units/g starch of isoamylase (EC 3.2.1.68), prepared from a culture of Pseudomonas amyloderamosa ATCC 21262, and 30 units/g starch of a soybean beta-amylase, saccharified at pH 5.0 for 36 hours, and purified in a manner similar to that described in Preparative Example 1 to obtain a high-purity maltose solution with a maltose content of 88.6%, which was then concentrated in vacuo into a syrup with a moisture content of 3.5%. The syrup was then transferred into a crystallizer, added with 2.5% crystalline alpha-maltose seed obtained by the method in Preparative Example 2, crystallized at 120°C for 10 minutes while stirring, poured into an aluminum tray, and aged at 70°C for 18 hours to obtain a solid. As described in Preparative Example 1, the solid was divided and dehydrated to obtain a pulverulent crystalline alpha-maltose with an alpha-maltose isomer content of 63.9% in a yield of about 94% based on the material high-purity maltose.

Preparative Example 4

A 45% aqueous solution of "HM-75", a starch sugar syrup with a maltose content of 79.6%, sold by Hayashibara Co., Ltd., Okayama, Japan, was used as the feed solution. "XT-1022 E (Na$^+$)", a strongly-acidic cation exchange resin, sold by Tokyo Chemical Industries, Kita-ku, Tokyo, Japan, was packed in water suspension in four jacketed stainless steel columns, 5.4 cm in diameter, to give respective bed depth of 5 m. The columns were cascaded to give a total bed depth of 20 m. The feed solution was admitted into the columns in an amount of 5 v/v % to the bed volume, and fractionated by passing 55°C water at a space velocity of 0.13 through the columns while keeping the inner temperature of the column at 55°C to obtain effluents. The maltose-rich fraction was separated from the effluents to obtain a high-purity maltose solution with a maltose content of 94.4%. After repeating these operations for twenty cycles, the resultant high-purity maltose solutions were pooled, and concentrated in vacuo to obtain a syrup with a moisture content of 4.0%, which was then transferred into a crystallizer, added with 2.0% crystalline alpha-maltose seed obtained by the method of Preparative Example 2, crystallized at 110°C for 20 minutes under stirring, and granulated with a screw-type extrusion granulator. The resultant product was then placed in a drying chamber, and aged therein by dehydration in a stream of 80°C air for 2 hours to obtain a pulverulent crystalline alpha-maltose with an alpha-maltose isomer content of 69.2% in a yield of about 93% based on the material high-purity maltose.

The following Examples illustrate the present invention in further detail.

Example 1

Maltose linoleate

Ten grams of the crystalline alpha-maltose powder, prepared by the method in Preparative Example 1, was suspended in 200 ml of dry pyridine and added with 4 g of thiasolithionelinoleic amide together with 5 ml of dry pyridine in an argon atmosphere at ambient temperature. Then, the mixture was added with 85 mg of sodium hydride in oil (60%) and allowed to react for 2 hours at ambient temperature. The reaction mixture was added with 1.5 ml of a saturated aqueous solution of ammonium chloride, and the pyridine was then removed by evaporation. The resultant solution was suspended in 200 ml of hot acetone, and the insoluble part was removed by filtration, after which the acetone was removed from the supernatant by distillation to obtain 8.5 g of a residue. The residue was purified by silica gel column chromatography to obtain 5.3 g of maltose linoleate.

The product is suitable for use as a tasteless, odourless, safe and highly-active non-ionic surfactant for foodstuffs, cosmetics, pharmaceuticals and chemicals.

As a control, a high-purity crystalline beta-maltose hydrate having a maltose content of 99.0% was treated in the same manner to obtain only 2.3 g of a strongly coloured maltose linoleate.

Example 2

Maltose myristate

Two hundred and twenty grams of a crystalline alpha-maltose powder prepared by the method in Preparative Example 2 was dissolved in 800 ml of N,N-dimethylformamide, added with 60 g of methyl myristate and 4 g of potassium carbonate, and heated to 85-95°C under a reduced pressure of 100-200 mmHg. After a lapse of 24 hours, the solvent was removed from the reaction mixture under reduced pressure, and the residue was soaked twice in 300 ml aliquots of acetone.

The residue was concentrated, and washed with benzene and petroleum ether after which the obtained oily residue was soaked in 300 ml of acetone.

The sediment obtained by ice-chilling the residue was separated from acetone, and dried to obtain 310 g of maltose myristate.

The product is suitable for use as a tasteless, odourless, safe and highly-active non-ionic surfactant for foodstuffs, cosmetics, pharmaceuticals and chemicals.

As a control, a high-purity crystalline beta-maltose hydrate having a maltose content of 99.0% was treated in the same manner to obtain only 90 g of a strongly coloured maltose myristate.

Example 3

Maltose dodecylate

Three hundred and ninety grams of n-dodecanol was placed in a reaction vessel and then heated to 125°C. The reaction vessel was added with 1 g of p-toluenesulfonic acid as the catalyst, and the pressure in the vessel was then reduced to 5-10 mmHg. Then, a homogeneous suspension containing 100 g of a crystalline alpha-maltose powder, prepared by the method in Preparative Example 3, and 130 g of n-dodecanol was added in the vessel at the rate of 2.3 g per minute over a period of 100 minutes. The reaction mixture was neutralized with a saturated solution of sodium bicarbonate, and the remaining alcohol was removed by evaporation to obtain a composition containing 2.2% of maltose and 79.9% of maltose dodecylate in the yield of 97.8%.

The product is suitable for use as a safe and active surfactant such as for laundry or kitchen detergent or shampoo.

As a control, a high-purity crystalline beta-maltose hydrate having a maltose content of 88.6% treated in the same manner only gave a composition containing 35% of maltose and 27.8% of a strongly coloured maltose dodecylate.

Example 4

Maltose laurate

A crystalline alpha-maltose prepared by the method in Preparative Example 2 was allowed to react in a manner similar to that described in Example 2, except that methyl myristate was replaced with methyl laurate, to obtain 260 g of maltose laurate.

The product is suitable for use in foodstuffs, cosmetics, pharmaceuticals and chemicals.

As a control, a high-purity crystalline beta-maltose hydrate having a maltose content of 94.4% was subjected to the same treatment; only 70 g of a coloured maltose laurate was obtained.

Example 5

Cyanuric derivative

Two grams of a crystalline alpha-maltose powder prepared by the method in Preparative Example 1 was suspended together with 5 w/v % of cyanuric chloride in 20 ml of N,N-dimethylformamide, and allowed to react for 3 hours at ambient temperature. The reaction mixture was filtered, and the resultant residue was washed with acetone, followed by drying.

The obtained cyanuric derivative of maltose was reacted with a highly-purified tetanus toxoid liquid in accordance with the method as disclosed in Japanese Patent Publication No. 8,090/82, and the reaction mixture was centrifugally separated to obtain a supernatant which was then subjected to ion exchange chromatography and gel filtration to recover the fraction of tetanus toxoid-maltose conjugate. The fraction was then filtered with a membrane filter to obtain a tetanus toxoid vaccine. The yield was about 85% against the toxin protein used.

As a control, a crystalline beta-maltose hydrate having a maltose content of 99.0% was treated in similar manner; the yield of tetanus toxoid vaccine was only about 5% against the toxin protein used.

As described above, according to the invention, high-purity maltose derivatvies, which are difficult to prepare from conventional anhydrous amorphous maltose or beta-maltose hydrate, can be prepared in high yield by allowing crystalline alpha-maltose to react with a suitable reagent under anhydrous conditions.

Because, unlike crystalline beta-maltose hydrate, crystalline alpha-maltose contains no water of crystallisation, the present invention shortens or, occasionally, even omits the drying step which is found to be essential when using anhydrous amorphous maltose and crystalline beta-maltose hydrate, the cost of producing maltose derivatives can be reduced.

Furthermore, the present invention diversifies the reaction of maltose because crystalline alpha-maltose is, due to its high affinity to various organic solvents, easily attacked by a reagent.

Therefore, the present invention enables low-cost industrial-scale production of high-quality maltose derivatives which are suitable for use, for example, in foodstuffs, biologically active substances, chemicals, synthetic intermediates, cosmetics and pharmaceuticals.

The present invention can be favourably practiced in analysis, wherein maltose must be converted into a derivative prior to the analytic step, to expedite the obtainment of accurate analytic data.

**Claims**

1.  A process for preparing a derivative of maltose, the process comprising reacting maltose with a suitable reagent under anhydrous conditions to form the desired derivative, characterised in that the said maltose is a crystalline alpha-maltose, whereby the desired derivative is produced in an almost theoretical yield.

2.  A process according to Claim 1, wherein the crystalline alpha-maltose contains at least 55 w/w % of alpha-maltose isomer based on the weight of the dry solid.

3.  A process according to Claim 1 or 2, wherein the crystalline alpha-maltose has been crystallized from a high-purity maltose having a maltose content of at least 85 w/w % based on the weight of the dry solid.

**4.** A process according to Claim 1, 2 or 3, wherein the maltose derivative obtained is an ester.

**5.** A process according to Claim 1, 2 or 3, wherein the maltose derivative obtained is an ether.

**Patentansprüche**

**1.** Verfahren zur Herstellung eines Maltosederivats, wobei das Verfahren die Reaktion von Maltose mit einem geeigneten Reagens unter wasserfreien Bedingungen umfaßt, um das gewünschte Derivat zu bilden, dadurch gekennzeichnet, daß die Maltose eine kristalline Alpha-Maltose ist, wodurch das gewünschte Derivat in nahezu theoretischer Ausbeute hergestellt wird.

**2.** Verfahren nach Anspruch 1, wobei die kristalline Alpha-Maltose wenigstens 55 Gew./Gew.-% eines Isomers der Alpha-Maltose enthält, bezogen auf das Gewicht der Trockensubstanz.

**3.** Verfahren nach Anspruch 1 oder 2, wobei die kristalline Alpha-Maltose aus einer Maltose hoher Reinheit mit einem Maltosegehalt von wenigstens 85 Gew/Gew.-% bezogen auf das Gewicht der Trockensubstanz, auskristallisiert wurde.

**4.** Verfahren nach Anspruch 1, 2 oder 3, wobei das erhaltene Maltosederivat ein Ester ist.

**5.** Verfahren nach Anspruch 1, 2 oder 3, wobei das erhaltene Maltosederivat ein Ether ist.

**Revendications**

**1.** Procédé pour la préparation d'un dérivé de maltose, le procédé comprenant la réaction du maltose avec un réactif approprié dans des conditions anhydres pour former le dérivé souhaité, caractérisé en ce que ledit maltose est un maltose alpha cristallin, par lequel le dérivé souhaité est produit en un rendement pondéral presque théorique.

**2.** Procédé selon la revendication 1, dans lequel le maltose alpha cristallin contient au moins 55% en poids d'isomère maltose alpha par rapport au poids de la matière sèche.

**3.** Procédé selon la revendication 1 ou 2, dans lequel le maltose alpha cristallin a été cristallisé à partir d'un maltose de grande pureté ayant une teneur en maltose d'au moins 85% par rapport au poids de la matière sèche.

**4.** Procédé selon la revendication 1, 2 ou 3, dans lequel le dérivé de maltose obtenu est un ester.

**5.** Procédé selon la revendication 1, 2 ou 3, dans lequel le dérivé de maltose obtenu est un éther.

FIG. 1

Diffraction angle (2θ°)

Intensity

FIG. 2

Diffraction angle (2θ°)

EP 0 225 774 B1

FIG. 3

FIG. 4

Intensity

Diffraction angle (2θ°)

EP 0 225 774 B1

FIG. 5

Diffraction angle (2θ°)

Intensity

FIG. 6

EP 0 225 774 B1